# EUROPEAN PATENT SPECIFICATION

(11) **EP 1 740 214 B1**
(45) Date of publication and mention of the grant of the patent: **03.10.2007**
(21) Application number: 05718419.4
(22) Date of filing: 11.04.2005
(51) Int. Cl.: A61K 47/12, A61K 47/14, A61K 31/545, A61K 31/546, A61K 9/19

(54) **METHOD OF STABILIZING DISORDERED CEFOVECIN SODIUM SALT**
VERFAHREN ZUR STABILISIERUNG VON UNGEORDNETEM CEFOVECIN-NATRIUMSALZ
PROCEDE DE STABILISATION DE SEL DE SODIUM DE CEFOVECINE DESORDONNE

(30) Priority: 22.04.2004 US 564372 P
(43) Date of publication of application: 10.01.2007
(73) Proprietor: Pfizer Products Incorporated, Groton, CT 06340 (US); University of Connecticut, Farmington, CT 06030-6207 (US)
(72) Inventor: PIKAL, Michael J, Farmington, CT06030-6207 (US); REDDY, Renuka Devi, Groton, CT 06340 (US); SHALAEV, Evgenyi Yur'evich, Groton, CT 06340 (US); ZIEGLER, Carl Bernard, Groton, CT 06340 (US)
(74) Representative: Rutt, Jason Edward
(86) International application number: PCT/IB2005/000955
(87) International publication number: WO 2005/102274

(56) References cited:
- WO-A-99/30688
- WO-A-03/053522
- WO-A-20/04082719
- WO-A-20/05009472

## Description

### FIELD OF THE INVENTION

The present invention relates to a method of stabilizing pharmaceutical compositions. In particular, the present invention relates to a method of increasing the stability of pharmaceutical compositions containing cefovecin sodium salt which is in an amorphous or other disordered state.

### BACKGROUND OF THE INVENTION

An understanding of the physical and chemical processes which can lead to unwanted changes in amorphous or other disordered state systems during prolonged storage is a fundamental concern to pharmaceutical scientists. Such changes can include chemical modifications and phase transformations which may affect various pharmaceutical properties such as stability and bioavailability of drug.

Freeze-drying or lyophilization is one well-known method used to stabilize drug substances. Freeze-drying may be accomplished by any method known in the art. Freeze-drying typically consists of three stages: 1) concentration of the product by freezing (removal of liquid water) 2) sublimation of the ice so formed; and 3) removal from the product of residual unfrozen water by diffusion/desorption/evaporation. Depending on its chemical composition and conditions of freeze-drying, the dried product may be wholly or partially disordered or amorphous. W003053522 describes ceforecin sodium salt its therapeutic uses, and standard compositions containing it.

WO99/30688 describes a method of lyophilizing a solution. The method comprises the steps of freezing the solution to a temperature at or below the lower of its eutectic temperature or its glass transition temperature, and in a first drying stage, removing at least a portion of the solvent by sublimation. The solution contains an accelerant excipient to enhance the rate of solvent sublimation.

While freeze-drying has become a standard method utilized for the stabilization of many drug substances in the solid state, many freeze-dried materials undergo chemical degradation during storage. Thus, there has been ongoing investigation to find ways to stabilize pharmaceutical compositions. The literature discloses annealing as one way to stabilize amorphous materials.

In a study made by S.V. Vasenkov, et al., "The influence of deep traps for gas molecules on oxygen transport in the glass of 2-methylpentanol-2", Chem. Phys. 195 (1995) 305-311, the effect of annealing slightly above Tg on oxidation of free radicals in 2-methylpentanol-2 was reported. The glassy sample was prepared by "freezing" of liquid 2-methylpentanol-2 in liquid nitrogen followed by gamma-irradiation. As shown in Figure 3 of the study paper, annealing at 166K, slightly above the Tg=163 K, increased the oxidation rate which could indicate increased instability. In a further study, B.V. Bol'shakov, et. al. discuss the effect of thermal annealing on oxidation of the tert-butyl radicals in butanol, "Formation of deep gas traps in glassy n-butanol", Phys. Chem. Chem. Phys. 2000, 2, 4793-4795. The glassy samples were prepared by "freezing" a solution that contained diphenylamin and tert-butylchloride in n-butanol in liquid nitrogen followed by UV irradiation. The thermal annealing was performed at temperatures above the Tg of the matrix, n-butanol.

S. Azarmi, et. al., in "Thermal treating as a tool for sustained release of indomethacin from Eudragit RS and RL matrices", Int. G. Pharm. 246 (2002) 171-177, describe how thermal treatment above the Tg was used to decrease a dissolution rate of indomethacin imbedded into an amorphous polymeric matrix. In this case, rate of dissolution was measured as a function of time and temperature of thermal treatment; chemical stability was not determined: The active agent, indomethacin, crystallized during thermal treatment.

U.S. 6,284,282 describes a method of preparing spray-freeze dried compositions for pulmonary administration. The compositions were prepared by atomizing liquid formulations containing proteins. The aqueous frozen solutions of proteins were subjected to annealing at sub-ambient temperatures to increase rate of subsequent sublimation.

M.J. Pikal in Peptide and Protein Delivery, 2nd ed. V.H. Less, Marcel Dekker, describes the lyophilized antibacterial, moxalactam (di-sodium salt), by annealing at 60°C (the annealing time was not specified). The amorphous moxalactam was heated at 60°C during the last stage of a freeze-drying (lyophilization) process. The stability of the annealed and control samples (no annealing) was measured during a stability study at 25 and 40°C by measuring the rate of decarboxylation of moxalactam (the duration of the stability study was not specified). The rate of decarboxylation was lower (i.e., stability is higher) in the annealed sample than in the control.

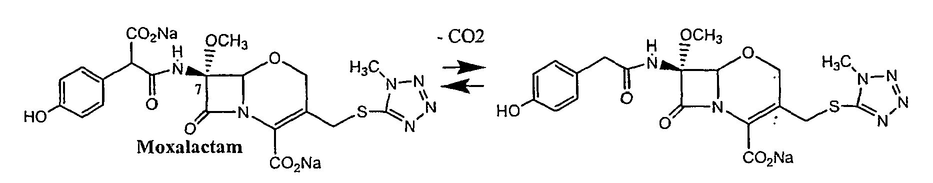

The literature does not teach how to determine optimal annealing conditions (i.e. choosing the optimum values of temperature and time). The only specific recommendation in the literature is that the annealing should be performed well below the glass transition temperature, Tg, of the amorphous material. In addition, it is noted that in the literature example annealing stabilized the amorphous moxalactam against the decarboxylation decomposition pathway. Decarboxylation of the 7-N-acyl side chain of moxalactam is a known degradation pathway and has been reported by Byrn in Pharmaceutical Research (1987), 4(2), 137-41.

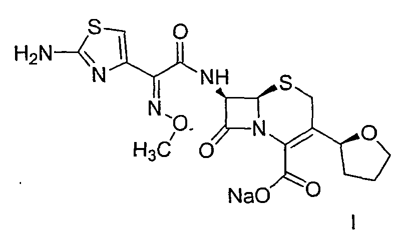

### SUMMARY OF THE INVENTION

According to one aspect, the present invention relates to a method for stabilizing a pharmaceutical composition comprising a compound of formula I 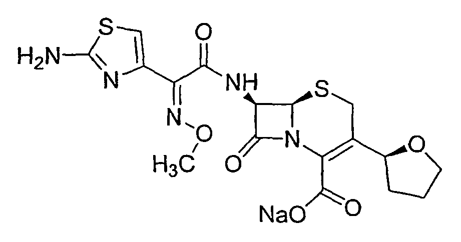 wherein the compound of formula I is in an amorphous or other disordered state and optionally one or more pharmaceutically acceptable excipients which comprises the steps of:
a) freeze-drying the composition until the composition has a residual solvent content which is less than about 5% by weight, based on the weight of the composition;
b) heating the composition to a temperature ranging from about 45°C to about 90°C;
c) maintaining the composition at the temperature the composition was heated to in step (b) for a period of time ranging from about one second to about 14 days; and
d) cooling the composition to a temperature that is below the temperature the composition was heated to in step (b).

According to another aspect, the present invention provides that the solvent of step (a) is water and the residual content of the water is less than 2 wt%.

In a further aspect, the heating temperature of step (b) is in a range of from about 55°C to about 80°C.

According to a further aspect of the present invention, the temperature of step (c) is maintained for a period of time ranging from about 5 minutes to about 40 hours.

In another aspect, the solvent of step (a) is water and the residual content of the water is less than 1 wt%.

According to yet another aspect, the heating temperature of step (b) is in a range of from about 60°C to about 75°C.

An additional aspect of the present invention provides the temperature of step (c) is maintained for a period of time ranging from about 20 minutes to about 10 hours.

According to a further aspect, the pharmaceutical composition further comprises one or more excipients selected from the group consisting of a buffer, a preservative, a bulking agent, and mixtures thereof.

In another aspect, the pharmaceutical composition further comprises citrate buffer, methyl paraben and propyl paraben.

### BRIEF DESCRIPTION OF THE DRAWINGS

Figure 1 is a graphical representation of storage stability of two samples treated according to the method of the present invention, and an untreated sample.
Figure 2 is a graphical representation of degradation rate constants of cefovecin sodium salt as a function of annealing time.

### DEFINITIONS

In the specification and claims that follow, reference will be made to a number of terms which shall be defined to have the following meanings:

The singular forms "a", "an" and "the" include plural referents unless the context clearly dictates otherwise.

"Optional" or "optionally" means that the subsequently described event or circumstance may or may not occur.

"Stabilize" as used herein may mean (i) the material has a longer shelf life, (ii) the material can be stored at a higher temperature for the same length of time as "non-stabilized" material, or (iii) the material experiences a lesser amount of chemical degradation or physical change when compared with "non-stabilized" material

"Treated material" refers to the active pharmaceutical agent which may optionally include pharmaceutically acceptable excipients that have been thermally treated or annealed.

"Excipients" as used herein refer to auxiliary ingredients, as known in the art, such as buffers, bulking agents, diluents, co-solvents, solvents, preservatives, tonicity adjusters, whose presence may help to provide a rapidly soluble freeze-dried product or extend the storage time of the formulation or satisfy special regulatory requirements.

The pharmaceutical material comprises the active drug and may also contain excipients and solvent that remains from production. The solvent may be an aqueous solvent, an organic solvent or a mixed aqueous/organic solvent. Examples of possible solvents and co-solvents are water and ethanol. Examples of preservatives are methylparaben and propylparaben. Typical buffers include phosphate, acetate, citrate, carbonate, and glycine.

The water-soluble bulking agent suitable for use in the present invention can be any of the pharmaceutically acceptable inert solid materials typically used for lyophilization. Such bulking agents include, for example, sugars such as glucose, maltose, sucrose and lactose; polyalcohols such as sorbitol and mannitol; amino acids such as glycine; polymers such as polyvinylpyrrolidone; polysaccharides such as dextran; certain inorganic salts such as sodium or potassium phosphates, or sodium chloride.

The ratio of the weight of the bulking agent to the weight of the compound of formula I used in the compositions of the present invention should generally be within the range of from about 0.01 to about 100, depending upon the bulking agent utilized. In a preferred embodiment, polyhydroxy compounds are the bulking agent of choice.

Amorphous solids or glasses are high energy quasi-solids that lack the long range order of the corresponding crystalline forms.

Amorphous materials are usually less chemically stable than crystalline materials. The chemical stability of amorphous materials may be improved by the methods of this invention.

"Other disordered state" refers to partially crystalline materials and crystalline mesophases with e.g. one-or two-dimensional translational order (liquid crystals), or orientational disorder (orientationally disordered crystals), or with conformational disorder (conformationally disordered crystals).

As used herein, the term "amorphous" includes those materials that may be present in some "other disordered state".

"Glass transition temperature" is represented by the symbol Tg and is the temperature at which an amorphous or disordered material changes from a brittle vitreous state to a plastic state.

Generally, Tg is determined using differential scanning calorimetry (DSC) and is standardly taken as the temperature at which onset of the change of heat capacity (Cp) of the composition occurs upon scanning through the transition. In the present specification, unless otherwise indicated, Tgs are determined by this method. Tg is dependent on composition and extent of annealing.

"Thermal treatment "or "annealing" refers to heating a pharmaceutical composition to a specified temperature, maintaining the temperature for a set period of time, and then cooling the composition.

"Residual solvent" refers to the liquid solvent that remains from prior processing steps, such as prior drying steps.

"Pharmaceutical compositions" refers to compositions that include at least one active pharmaceutical agent, and can include one or more pharmaceutically acceptable excipients. The terms "pharmaceutical compositions," "pharmaceutical material" and "pharmaceutical formulation" are used interchangeably in the present specification.

Formulations of the compound of formula I can be isolated by drying, preferably by lyophilization as known in the art. Usually the lyophile formulations are produced with ampule lyophilization, vial lyophilization, tray lyophilization, or like conventional methods by cooling the formulations at subzero temperature to freezing. The frozen material is then dried under vacuum by subliming the water component originally contained in the solution as a solvent, thus leaving a solid lyophilized cake. Thus, for example, the excipients described above and the compound of formula I are successively dissolved under stirring in a suitable amount of water for injections. Further water is added to reach the desired final volume. The resulting solution is clarified, sterile filtered and aseptically distributed in sterile containers (*e.g*. vials) of desired capacity. Freeze-drying the solution is then performed and the vials are hermetically sealed according to conventional procedures.

The lyophilized drug product is the compound of formula I, in an amorphous or other disordered state. When a product solution is required, it can be reconstituted by dissolving the dry formulation in water for injection, bacteriostatic water for injection or another pharmaceutically acceptable diluent (*e.g*. isotonic solution of sodium chloride, water for injection with ethanol or citrate buffer, and bacteriostatic water for injection with benzyl alcohol) in an amount sufficient to generate a solution of the required strength for parenteral administration to patients.

### DETAILED DESCRIPTION OF THE INVENTION

The present inventors have determined novel annealing conditions to increase the chemical stability of cefovecin sodium salt of formula I which is in an amorphous or other disordered state.

Amorphous lyophilized cefovecin sodium salt was annealed at four different temperatures, 60°C, 75°C, 160°C, and 170°C. The inventors found that annealing at 60°C and 75°C increased the stability of the compound whereas annealing at 160°C and 170°C resulted in unacceptable levels of degradation.

In one embodiment of the present invention, the pharmaceutical composition comprises from about 0.1 wt% to about 100 wt% of a compound of formula I 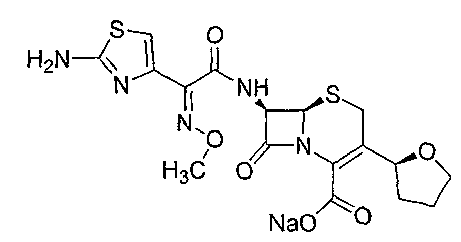 wherein the compound of formula I is in an amorphous or other disordered state

The pharmaceutical composition of the present invention comprises less than about 5%, preferably less than about 2% and most preferably less than about 1% by weight of residual solvent, based on the weight of the pharmaceutical composition (formulation). Most preferably, the solvent is water.

In one aspect of the present invention, the pharmaceutical materials are subjected to a freeze drying step prior to thermal treatment. The material may be "pre-treated", i.e. the material is subjected to processing and is stored before the thermal treatment step or the material is subjected to processing immediately before the thermal treatment step.

During conditions of freeze drying and thermal treatment, the active pharmaceutical agent must be in a partially or completely glassy or amorphous form.

In the present invention, crystallization, i.e. formation of a highly ordered structure, did not occur during thermal treatment; the material remained in the amorphous form. Applicants herein unexpectedly discovered that thermal treatment performed according to the present invention increased chemical stability of amorphous cefovecin sodium salt without causing crystallization of the active agent.

According to a preferred aspect of the present invention, the pharmaceutically active agent is a cephalosporin compound or its pharmaceutically acceptable salt. Cephalosporins are widely used antibiotics. The cephalosporin compounds which may be treated according to the methods of the present invention are those disclosed in U.S. Patent Numbers 6,001,997, 6,020,329 and 6,077,952.

The cephalosporin compounds of Formula I, below, are broad spectrum cephalosporin antibacterials, and are used in the treatment of bacterial infections in animals. Pharmaceutical compositions comprising alkali metal salts of the compound of formula I and optional pharmaceutically acceptable excipients may also be stablilized according to the methods of the present invention. In one embodiment, the pharmaceutically active agent is (6R,7R)-7-[[(2Z)-(2-amino-4-thiazolyl)(methoxyimino)acetyl]amino]-8-oxo-3-[2(S)-tetrahydro-2-furanyl]-5-thia-1-azaabicyclo[4.2.0]oct-2-ene-2-carboxylic acid, monosodium salt. The compound of formula is referred to herein as "cefovecin sodium salt".

Cephalosporin compounds of Formula I may comprise up to about 100% of the treated material. In other cases, depending on any particular dosage form requirement, a buffer, preservatives, and other pharmaceutically acceptable excipients may be used

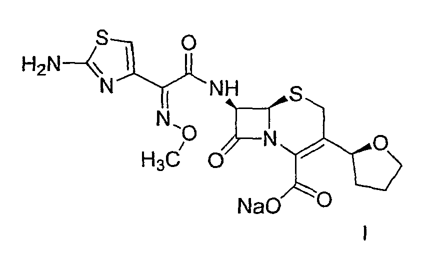

The particular excipients used in the treated material will depend on the intended form of administration. Appropriate excipients for each intended form of administration are known in the art. In one embodiment of this invention the treated material is in injectable form. Suitable excipients for injectable products are well-known to skilled individuals and include, but are not limited to, preservatives, stabilizers, emulsifiers, solubilizers, antioxidants, buffers, bulking agents, solvents and tonicity agents. See also, for example, Handbook of Pharmaceutical Excipients, Third ed., A.H. Kibbe (Ed.), APhA, Washington, D.C., 2000.

In one example of an embodiment of the present invention, the treated material comprises a cephalosporin compound of formula (I) and, optionally a solvent such as water. The compound of formula I may be dissolved in water at a concentration of from about 5 to about 300 mg/ml, preferably from about 5 to about 200 mg/ml, sterilized using sterilizing filtration, filled in glass vials, and freeze-dried. In such aspect of the invention, the temperature of the water/cephalosporin mixture is reduced, in a primary drying step, to about -20°C to about -40°C, and a pressure of about 6.67 Pa to about 53.33 Pa (about 50 to about 400 millitorrs). At these conditions, the water in the mixture is sublimated. The primary drying step is followed by a secondary drying step at about 20°C to about 40°C under about 6.67 Pa to about 53.33 Pa (about 50 to about 400 millitorrs). It is preferable to reduce the water content to about 1.0% by weight or less. Afterwards, the temperature is increased above the Tg and thermal treatment may be effected.

In the present invention, it is expected that materials treated by the methods as described herein are more stable and not subject to degradation by hydrolysis, dimerization, isomerization, and other chemical mechanisms, and/or have increased physical stability, as compared to non-treated materials.

### EXAMPLES

### Example 1. Annealing at 75°C

Compound of formula I, cefovecin sodium salt, was obtained from ACS DobFar (Italy). Water for Injections (WFI) was obtained from a commercial source B-Braun. 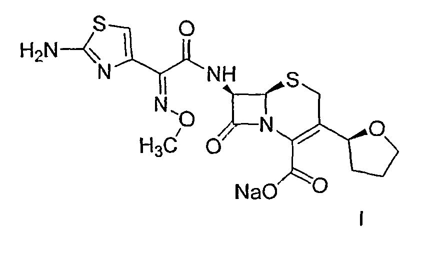 Cefovecin sodium salt was dissolved in WFI at a concentration of 200 mg cefovecin sodium salt/ml. This solution was filtered using 0.22µ sterilized filters. 4.25 ml of this solution was volumetrically filled in the laminar flow hood into a 20-ml Flint Type I Tubular Treated Vials. 20mm Lyophile D777-1, B2TR FluroTec Single Vent Stoppers were partially inserted in the vials. The vials were lyophilized using Vertis freeze dryer (Gardiner, New York) using the following cycle: frozen at -40°C followed by vacuum drying at the shelf temperature of -18°C for approximately 42 hours, and 8.00 Pa (60 millitore) vacuum; secondary dried at a temperature of 40°C for approximately 12 hours and 8.00 Pa (60 millitore) vacuum. The lyophilizer chamber was back flushed with nitrogen and the vials were capped and sealed with aluminum shells. The lyophilization process produced amorphous cefovecin sodium salt.

Amorphous lyophilized cefovecin sodium salt was annealed in sealed vials at 75°C for 20 and 60 min. The sealed vials with untreated cefovecin sodium salt (control), and vials with annealed cefovecin sodium salt were stored at 40°C at ambient humidity for 6 and 12 weeks.

Reverse Phase High Performance Liquid Chromatography (RP-HPLC) was used to monitor degradation of cefovecin sodium salt. HPLC was carried out with a Waters Alliance (Milford, MA) system with a UV detector set at 256 nm and a Kromasil C₄ column 4.6X 250 mm, 5 µm, 10 nm (100 angstrom) pore size (Waters, Milford, MA). Column temperature was 30 ± 2°C and the samples were maintained in the auto sampler at 5 ± 3°C and the flow rate was 1 ml/min. Gradient method was used with mobile phase A consisting of (9:1) 0.025M sodium phosphate buffer solution, pH 6.5: acetonitrile in HPLC grade water, and mobile phase B consisting of (4:6) 0.025M sodium phosphate buffer solution, pH 6.5: acetonitrile in HPLC grade water.

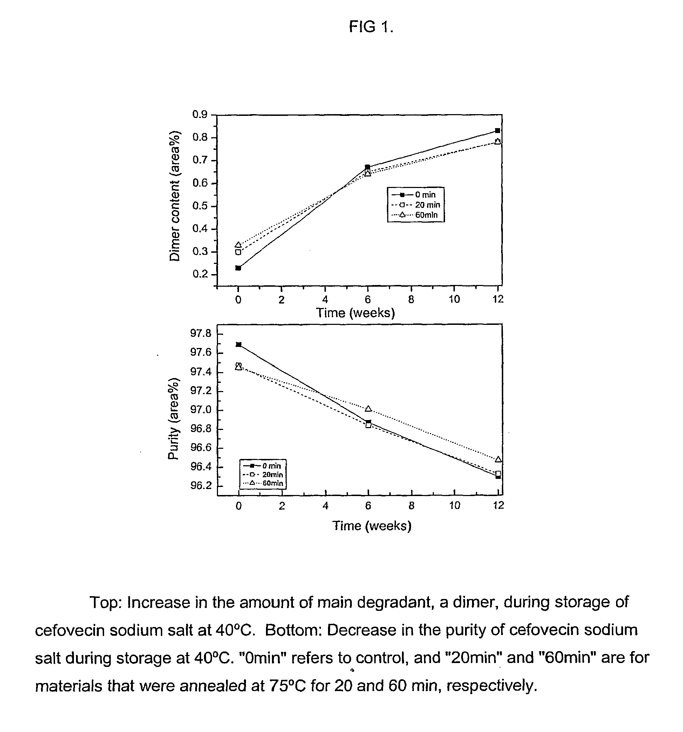

The lyophilized cake was dissolved in WFI, and the solution was injected into the HPLC column. Contents of cefovecin sodium salt and a main degradant with relative retention time of approximately 1.6 were calculated as A_{c}/ΣAᵢ, where A_{c} is the peak area of either cefovecin sodium salt or the degradant, and ΣAᵢ is a total area of all chromatographic peaks. This degradant was tentatively identified as a dimer. Stability data obtained for two annealed samples and the control sample are graphically illustrated in Figure 1. It can be seen that the annealing at 20 and 60 min (at 75°C) resulted in some degradation (Fig. 1, initial time points) as expressed in lower purity (bottom) and higher level of dimer (top). However, after storage for 12 weeks at 40°C at ambient humidity, overall purity was higher, and the dimer level were lower in the annealed samples, comparing with control. This is indicative of a higher stability of annealed samples.

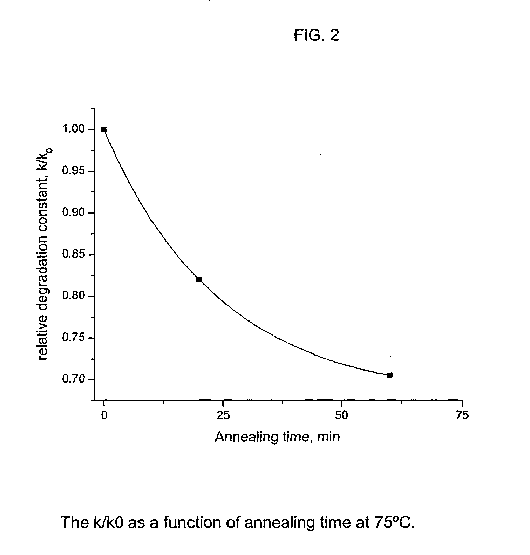

In order to express stabilization by annealing in a more quantitative term, degradation rate constants were determined from the data of loss of cefovecin sodium salt (Fig. 1, bottom) using a zero-order kinetic model. The rate constants, k, are given in Table 1. It can be seen that the k for annealed samples were lower than that for the control (no annealing). In addition, relative rate constants, k/k0 (where k0 is the rate constant of the control) were calculated in relation to control. The k/k0 are plotted in Fig. 2 as a function of annealing time at 75°C.

Hence, the higher purity and lower level of dimer at the 12 week time point (Fig. 1), and a lower rate of degradation in the annealed materials (Table 1, Fig. 2) demonstrate that annealing increased the thermal stability of cefovecin sodium salt.

**Table 1. Degradation rate constants of cefovecin sodium salt at 40°C. Numbers in () are standard deviation.**

| Annealing conditions, time at 75°C | k, %/week | k/k0 |
|---|---|---|
| 0 min (control) | -0.12(0.01) | 1.00 |
| 20 min | -0.095 (0.006) | 0.82 |
| 60 min | -0.082 (0.005) | 0.71 |

### Example 2. Annealing at 60°C.

Amorphous lyophilized cefovecin sodium salt was annealed in sealed vials at 60°C for 300 min in a contract lab, UCONN, Storrs, CT. Untreated samples (control), and annealed samples were placed on stability at 40°C for 6 months. Reverse Phase High Performance Liquid Chromatography was used to monitor degradation as described in Example 1.

After storage for 6 months at 40°C, the purity of the annealed sample was higher whereas dimer level was lower than that of the control (Table 2). Higher purity and lower level of dimer (Table 2) indicated that annealing at 60°C decreased degradation rate and stabilized amorphous cefovecin sodium salt.

**Table 2. Purity of amorphous freeze-dried cefovecin sodium salt during storage at 40°C.**

| Time at 40°C | purity (cefovecin sodium salt), area% | | degradation product, dimer, area% | |
|---|---|---|---|---|
| (months) | control | annealed | control | annealed |
| 0 | 98.35 | 98.11 | 0.17 | 0.34 |
| 6 | 96.35 | 96.49 | 0.96 | 0.93 |

### Example 3. Annealing at 160°C/120 min and 170°C/45 min.

A freeze-dried formulation that contains cefovecin sodium salt was prepared as follows. Cefovecin sodium salt was dissolved in citrate buffer at pH 6.7. Two inactive ingredients (preservatives), methylparaben and propylparaben, were added after dissolution of cefovecin sodium salt was visually completed. The solution contained 200 mg/ml of cefovecin sodium salt, 50 mM citrate buffer, 4.5 mg/ml methylparaben, and 0.5 mg/ml propylparaben. The solution was filled in 20 ml glass vials, 4.25 ml/vial. The vials were partially stoppered with single-vent D777-1 and loaded into a freeze-drier (Hull 450 FXS800S, Warminster, PA). The solution was frozen at -40°C followed by primary drying at the shelf temperature of -10°C and approximately 26.67 Pa (200 millitore) vacuum; after the product temperature approached -12°C, secondary drying was performed at shelf temperature 40°C for approximately 12 hours and 26.67 Pa (200 millitore) vacuum. Lyophilized cefovecin sodium salt was prepared in a contract facility, SmithKline Beecham Corporation, Conshocken, PA. The lyophilized samples had water content of approximately 0.3 wt%. The lyophilized samples were annealed in sealed vials at 160°C for 120 min and 170°C/45 min. The untreated samples (control), and annealed samples were tested by HPLC; HPLC conditions are described in Example 1. The purity results are given in Table 3. It can be seen that the treatment at both 160°C and 170°C resulted in significant degradation comparing with examples 1 and 2.

**Table 3. Purity results of thermally treated freeze-dried cefovecin sodium salt.**

| Annealing conditions | Purity (area %) | Comment |
|---|---|---|
| 160°C/120 minutes | 69.8 | Example 3 |
| 170°C /45 minutes | 70.3 | Example 3 |
| 60°C/300 min | 98.1 | Example 2 |
| 75°C/20 min | 97.5 | Example 1 |
| 75°C/60 min | 97.5 | Example 1 |

## Claims

1. A method for stabilizing a pharmaceutical composition comprising a compound of formula I 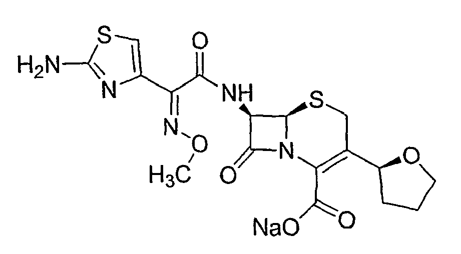 wherein the compound of formula I is in an amorphous state
and optionally one or more pharmaceutically acceptable excipients which comprises the steps of:
a) freeze-drying the composition until the composition has a residual solvent content which is less than about 5% by weight, based on the weight of the composition;
b) heating the composition to a temperature ranging from about 45°C to about 90°C;
c) maintaining the composition at the temperature the composition was heated to in step (b) for a period of time ranging from about one second to about 14 days; and
d) cooling the composition to a temperature that is below the temperature the composition was heated to in step (b).

2. The method according to claim 1 wherein the solvent is water and the residual content of the water is less than 2 wt%.

3. The method according to claim 1 wherein the heating temperature of step (b) is in a range of from about 55°C to about 80°C.

4. The method according to claim 1, wherein the temperature of step (c) is maintained for a period of time ranging from about 5 minutes to about 40 hours.

5. The method according to claim 1 wherein the solvent is water and the residual content of the water is less than 1 wt%.

6. The method according to claim 1 wherein the heating temperature of step (b) is in a range of from about 60°C to about 75°C.

7. The method according to claim 1, wherein the temperature of step (c) is maintained for a period of time ranging from about 20 minutes to about 10 hours.

8. The method according to claim 1, wherein the pharmaceutical composition further comprises one or more excipients selected from the group consisting of a buffer, a preservative, a bulking agent, and mixtures thereof.

9. The method according to claim 8 wherein the pharmaceutical composition further comprises citrate buffer, methyl paraben and propyl paraben.

10. A pharmaceutical composition comprising a compound of formula I 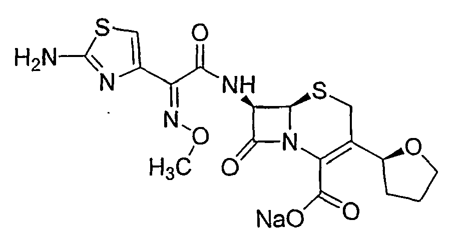 wherein the compound of formula I is in an amorphous state, prepared by the method of claim 1.

11. The pharmaceutical composition according to claim 10 further comprising citrate buffer, methyl paraben and propyl paraben.

## Patentansprüche

1. Verfahren zum Stabilisieren einer pharmazeutischen. Zusammensetzung, umfassend eine Verbindung der Formel I 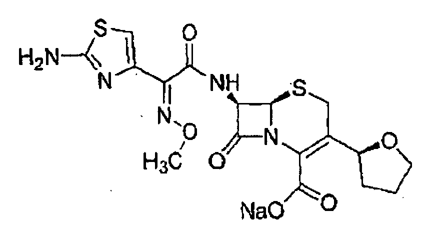 wobei die Verbindung der Formel I in einem amorphen Zustand vorliegt, und gegebenenfalls einen oder mehrere pharmazeutisch verträgliche Exzipienten, das die Schritte umfasst von:
a) Gefriertrocknen der Zusammensetzung, bis die Zusammensetzung einen Restlösungsmittelgehalt aufweist, der weniger als etwa 5 Gewichtsprozent ist, bezogen auf das Gewicht der Zusammensetzung;
b) Erhitzen der Zusammensetzung auf eine Temperatur im Bereich von etwa 45°C bis etwa 90°C;
c) Halten der Zusammensetzung bei der Temperatur, auf die die Zusammensetzung in Schritt (b) erhitzt wurde, für einen Zeitraum im Bereich von etwa einer Sekunde bis etwa 14 Tage; und
d) Kühlen der Zusammensetzung auf eine Temperatur, die unterhalb der Temperatur liegt, auf die die Zusammensetzung in Schritt (b) erhitzt-wurde.

2. Verfahren nach Anspruch 1, wobei das Lösungsmittel Wasser ist und der restliche Wassergehalt weniger als 2 Gewichtsprozent ist.

3. Verfahren nach Anspruch 1, wobei die Heiztemperatur von Schritt (b) in einem Bereich von etwa 55°C bis etwa 80°C liegt.

4. Verfahren nach Anspruch 1, wobei die Temperatur von Schritt (c) für einen Zeitraum im Bereich von etwa 5 Minuten bis etwa 40 Stunden gehalten wird.

5. Verfahren nach Anspruch 1, wobei das Lösungsmittel Wasser ist und der restliche Wassergehalt weniger als 1 Gewichtsprozent ist.

6. Verfahren nach Anspruch 1, wobei die Heiztemperatur von Schritt (b) in einem Bereich von etwa 60°C bis etwa 75°C liegt.

7. Verfahren nach Anspruch 1, wobei die Temperatur von Schritt (c) für einen Zeitraum im Bereich von etwa 20 Minuten bis etwa 10 Stunden gehalten wird.

8. Verfahren nach Anspruch 1, wobei die pharmazeutische Zusammensetzung weiterhin einen oder mehrere Exzipienten, ausgewählt aus der Gruppe, bestehend aus einem Puffer, einem Konservierungsmittel, einem Füllmittel und Gemischen davon, umfasst.

9. Verfahren nach Anspruch 8, wobei die pharmazeutische Zusammensetzung weiterhin Citratpuffer, Methylparaben und Propylparaben umfasst.

10. Pharmazeutische Zusammensetzung, umfassend eine Verbindung der Formel I 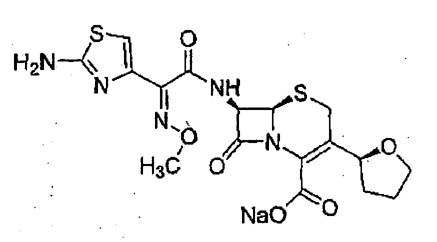 wobei die Verbindung von Formel I in einem amorphen Zustand vorliegt, hergestellt durch das Verfahren nach Anspruch 1,

11. Pharmazeutische Zusammensetzung nach Anspruch 10, weiterhin umfassend Citratpuffer, Methylparaben und Propylparaben.

## Revendications

1. Procédé pour stabiliser une composition pharmaceutique comprenant un composé de formule I 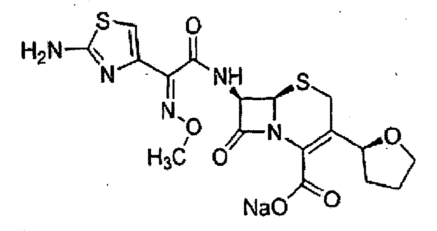 dans laquelle le composé de formule I est à l'état amorphe,
et facultativement un ou plusieurs excipients pharmaceutiquement acceptables, qui comprend les étapes consistant à :
a) lyophiliser la composition jusqu'à ce que la composition ait une teneur en solvant résiduel qui soit inférieure à environ 5 % en poids, sur la base du poids de la composition ;
b) à chauffer la composition à une température allant d'environ 45°C à environ 90°C ;
c) à maintenir la composition à la température à laquelle la composition a été chauffée dans l'étape b) pendant une période de temps allant d'environ une seconde à environ 14 jours ; et
d) à refroidir la composition à une température qui est inférieure à la température à laquelle la composition a été chauffée dans l'étape b).

2. Procédé suivant la revendication 1, dans lequel le solvant est l'eau et la teneur résiduelle en eau est inférieure à 2 % en poids.

3. Procédé suivant la revendication 1, dans lequel la température de chauffage de l'étape b) est comprise dans l'intervalle d'environ 55°C à environ 80°C.

4. Procédé suivant la revendication 1, dans lequel la température de l'étape c) est maintenue pendant une période de temps allant d'environ 5 minutes à environ 40 heures.

5. Procédé suivant la revendication 1, dans lequel le solvant est l'eau et la teneur résiduelle en eau est inférieure à 1 % en poids.

6. Procédé suivant la revendication 1, dans lequel la température de chauffage de l'étape b) est comprise dans l'intervalle d'environ 60°C à environ 75°C.

7. Procédé suivant la revendication 1, dans lequel la température de l'étape c) est maintenue pendant une période de temps allant d'environ 20 minutes à environ 10 heures.

8. Procédé suivant la revendication 1, dans lequel la composition pharmaceutique comprend en outre un ou plusieurs excipients choisis dans le groupe consistant en un tampon, un conservateur, une charge et leurs mélanges.

9. Procédé suivant la revendication 8, dans lequel la composition pharmaceutique comprend en outre un tampon au citrate, du parahydroxybenzoate de méthyle et du parahydroxybenzoate de propyle.

10. Composition pharmaceutique comprenant un composé de formule I 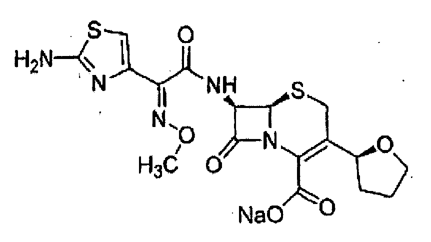 dans laquelle le composé de formule I est à l'état amorphe, préparée par le procédé de la revendication 1.

11. Composition pharmaceutique suivant la revendication 10, comprenant en outre un tampon au citrate, du parahydroxybenzoate de méthyle et du parahydroxybenzoate de propyle.
